# EUROPEAN PATENT APPLICATION

(11) **EP 2 824 632 A1**
(43) Date of publication of application: **14.01.2015**
(21) Application number: 12874348.1
(22) Date of filing: 09.10.2012
(51) Int. Cl.: G06Q 50/22

(54) **HEALTH INFORMATION SYSTEM**

(30) Priority: 10.04.2012 CN 201210102361
(71) Applicant: Huawei Technologies Co., Ltd, Shenzhen, Guangdong 518129 (CN)
(72) Inventor: WEN, Changcheng, Shenzhen Guangdong 518129 (CN); LIU, Bo, Shenzhen Guangdong 518129 (CN)
(74) Representative: Körber, Martin Hans
(86) International application number: PCT/CN2012/082643
(87) International publication number: WO 2013/152579

(57) **Abstract**

The present invention discloses a health information system and belongs to the field of data management. The system includes: at least one medical institution server, a front end processor, two or more data gateways, and a data exchange platform, where the at least one medical institution server is configured to send medical data to the front end processor; the front end processor is configured to receive the medical data from the at least one medical institution server, acquire a destination data gateway from the two or more data gateways, and send the medical data to the destination data gateway; the two or more data gateways are configured to receive the medical data and send the medical data to the data exchange platform; and the data exchange platform is configured to process the received medical data. The present invention prevents a service from being affected due to congestion of a single data gateway in busy hours or a service from being affected due to a fault of a front end processor of a medical institution, thereby improving data stability and ensuring a normal medical service.

## Description

### CROSS-REFERENCE TO RELATED APPLICATIONS

This application claims priority to Chinese Patent Application No. 201210102361.8, filed with the Chinese Patent Office on April 10, 2012, and entitled "HEALTH INFORMATION SYSTEM", which is incorporated herein by reference in its entirety.

### TECHNICAL FIELD

The present invention relates to the field of data management, and in particular, to a health information system.

### BACKGROUND

A regional health information platform is a platform for medical institutions in a region to save and exchange medical data. The regional health information platform is generally established within a county, a city, a province, or a country in a unified manner, and is managed and maintained by a government or another neutral institution. The regional health information platform interworks with an information system of a medical institution by using a front end data processor. The front end data processor is located between the regional health information platform and the information system of the medical institution, is near to the information system of the medical institution, and collaborates on information transfer in these two directions. The regional health information platform acquires data from the information system of the medical institution by using a front end medical data processor; and a front end data processor of a medical institution acquires data from a health information platform. The regional health information platform saves health data of medical institutions in a region, implements medical data exchange between the medical institutions, and is also responsible for exchanging data with an upper-level platform. Due to existence of a regional medical information platform, data that is collected by one party is shared among multiple parties and a collaboration of medical service is achieved.

In a process of implementing the present invention, the inventor finds that the prior art at least has the following problems: A data gateway of a regional health information platform connects information systems of all medical institutions which are in the charge of the data gateway of the regional health information platform. When the data gateway or a front end processor has a fault, an information system of a medical institution, which is connected to the data gateway or the front end processor, loses data contact with the regional health information platform, so that data stability is poor. Once a fault occurs, a medical institution cannot perform data exchange with the regional health information platform, thereby seriously affecting a normal medical service.

### SUMMARY

To improve data stability, embodiments of the present invention provide a health information system. The technical solutions are as follows:

A health information system, where the system includes at least one medical institution server, a front end processor, two or more data gateways, and a data exchange platform, where:
the at least one medical institution server is configured to send medical data to the front end processor;
the front end processor is configured to receive the medical data from the at least one medical institution server, acquire a destination data gateway from the two or more data gateways, and send the medical data to the destination data gateway;
the two or more data gateways are configured to receive the medical data and send the medical data to the data exchange platform; and
the data exchange platform is configured to process the received medical data.

A health information system, where the system includes at least one medical institution server, two or more front end processors, a data gateway, and a data exchange platform, where:
the at least one medical institution server is configured to acquire a destination front end processor from the two or more front end processors and send medical data to the destination front end processor;
the two or more front end processors are configured to receive the medical data from the at least one medical institution server and send the medical data to the data gateway;
the data gateway is configured to receive the medical data and send the medical data to the data exchange platform; and
the data exchange platform is configured to process the received medical data.

The technical solutions provided by embodiments of the present invention bring the following beneficial effects: Multiple data gateways or multiple front end processors are disposed in a health information system, so that the health information system is capable of supporting multiple connections between the front end processors and the data gateways, thereby preventing a service from being affected due to congestion of a single data gateway in busy hours or a service from being affected due to a fault on a front end processor of a medical institution, improving data stability, and ensuring a normal medical service.

### BRIEF DESCRIPTION OF DRAWINGS

To describe the technical solutions in the embodiments of the present invention more clearly, the following briefly introduces the accompanying drawings required for describing the embodiments. Apparently, the accompanying drawings in the following description show merely some embodiments of the present invention, and a person of ordinary skill in the art may still derive other drawings from these accompanying drawings without creative efforts.
FIG. 1 is a schematic structural diagram of a health information system according to an embodiment of the present invention;
FIG. 2 is a schematic structural diagram of a health information system according to an embodiment of the present invention;
FIG. 3 is a schematic structural diagram of a front end processor according to an embodiment of the present invention;
FIG. 4 is a schematic structural diagram of a health information system according to an embodiment of the present invention;
FIG. 5 is a schematic structural diagram of a health information system according to an embodiment of the present invention;
FIG. 6 is a schematic structural diagram of a medical institution server according to an embodiment of the present invention; and
FIG. 7 is a schematic organizational diagram of a health information system according to an embodiment of the present invention.

### DESCRIPTION OF EMBODIMENTS

To make the objectives, technical solutions, and advantages of the present invention clearer, the following further describes the embodiments of the present invention in detail with reference to the accompanying drawings.

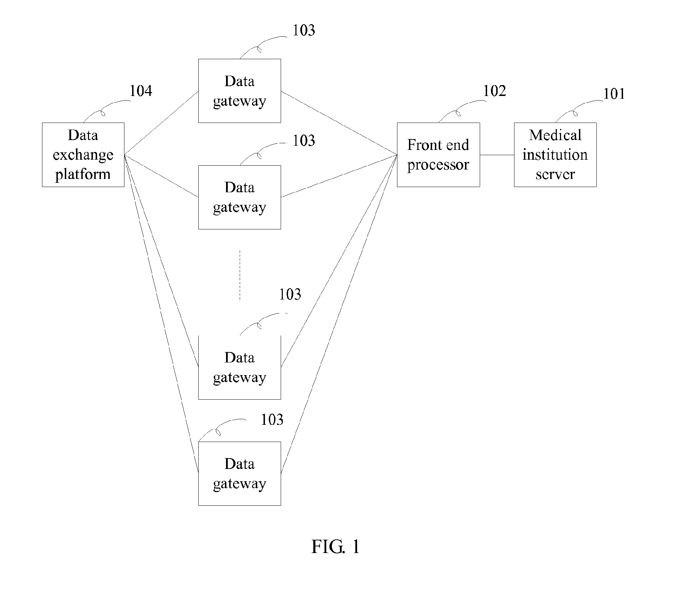

FIG. 1 is a schematic structural diagram of a health information system according to an embodiment of the present invention. Referring to FIG. 1, the system includes at least one medical institution server 101, a front end processor 102, two or more data gateways 103, and a data exchange platform 104.

(1) The at least one medical institution server 101 is configured to send medical data to the front end processor.

Specifically, the medical institution server 101 includes a database system and a service system of a medical institution. The database system is configured to store medical data generated in a medical service, for example, basic patient information, electronic medical record information, inspection and check information, and medical order information. The service system may provide an information service for the medical institution based on the database system. The service system may include but is not limited to a hospital information system, a clinical information system, an electronic medical record system, a medical imaging system, and the like.

(2) The front end processor 102 is configured to receive the medical data from the at least one medical institution server, acquire a destination data gateway from the two or more data gateways, and send the medical data to the destination data gateway.

The front end processor is configured to solve a problem of interworking between the data exchange platform and the medical institution server. On one hand, the front end processor accesses the data exchange platform through the data gateway. On the other hand, the front end processor accesses the medical institution server, communicates with the data exchange platform and the medical institution server, and performs data conversion.

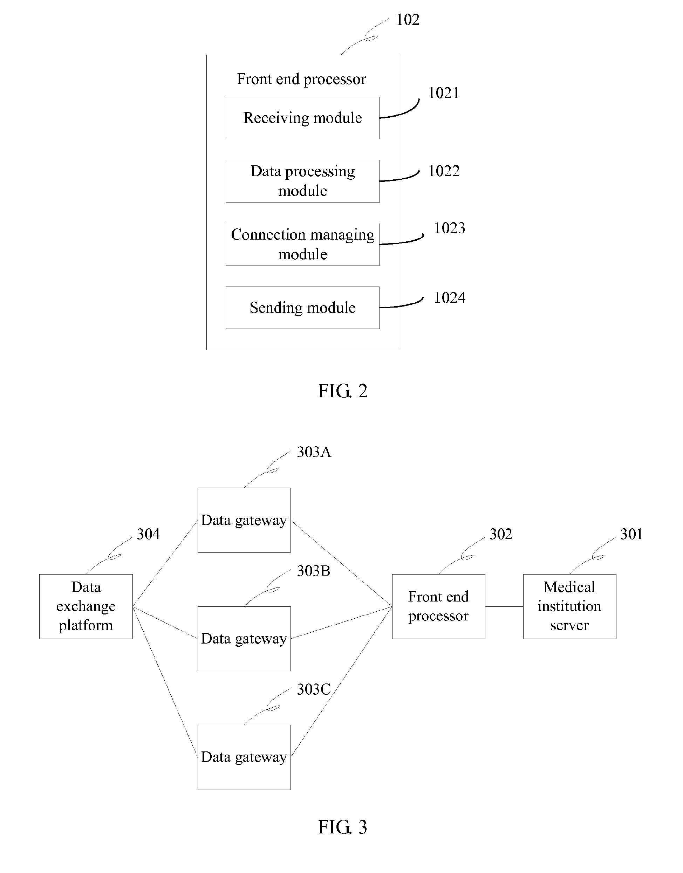

Preferably, referring to FIG. 2, the front end processor 102 includes: a receiving module 1021, a data processing module 1022, a connection managing module 1023, and a sending module 1024. The receiving module 1021 is configured to receive the medical data from the at least one medical institution server. The data processing module 1022 is configured to convert a data format of the medical data into a data format required by the data exchange platform. The connection managing module 1023 is configured to acquire the destination data gateway from the two or more data gateways and establish a connection between the front end processor and the destination data gateway. The sending module 1024 is configured to send the medical data to the destination data gateway through the established connection.

The data processing module 102 is specifically configured to extract the medical data from a received data packet piece by piece, parse the data format of the medical data from an external protocol into an internal data packet format, and convert the internal data packet format into an external protocol format. The external protocol format is the data format required by the data exchange platform.

The connection managing module 1023 is specifically configured to be responsible for establishing a connection to a data gateway of a regional health information platform and managing a connection state. The connection may use an IP, TCP, UDP, and WebService technology, and the like.

Preferably, the connection managing module 1023 specifically includes: a destination data gateway determining unit, configured to acquire, according to a first preset rule, one or more data gateway from the two or more data gateways as the destination data gateway; and a connection establishing unit, configured to establish a connection to the destination data gateway. It should be noted that a front end processor may use multiple methods to determine a destination data gateway and a specific method is set by a technician according to a system requirement, network bandwidth, or the like. Preferably, any one of the following destination data gateway determining units may be available:

(a) The destination data gateway determining unit is specifically configured to randomly acquire one data gateway from the two or more data gateways as the destination data gateway.

(b) The destination data gateway determining unit is specifically configured to acquire, according to a preset sequence, one data gateway from the two or more data gateways as the destination data gateway.

(c) The destination data gateway determining unit is specifically configured to use the two or more data gateways as the destination data gateway.

(d) The destination data gateway determining unit is specifically configured to determine whether an active data gateway in the two or more data gateways runs normally; and acquire the active data gateway as the destination data gateway if the active data gateway runs normally; and acquire a standby data gateway in the two or more data gateways as the destination data gateway if the active data gateway runs abnormally.

Preferably, when the number of determined destination data gateways is two or more, the sending module is specifically configured to send the medical data to each destination data gateway or any one of the two or more destination data gateways. The medical data may be sent randomly or according to a manner of alternately sharing information traffic, that is, data transferred between the data exchange platform and the medical institution is sent on multiple links according to a random sequence, an alternate sequence, or the like.

The sending module 1024 is specifically configured to classify the medical data according to a user identifier of each piece of medical data and send a same type of medical data to a same destination data gateway. This sending manner is to share information traffic based on the user identifier of the medical data. Health information is mostly classified according to a residential region and medical data with a same user identifier may be regarded as the same type of medical data. Therefore, medical data including a same user identifier or a same type of user identifier is transmitted fixedly through one of multiple connections, so as to implement traffic sharing.

For a health information system including two or more data gateways, a front end processor may establish connections to the two or more data gateways. In this way, two or more data connections exist in the health information system. The two or more data connections may carry medical data by using a load sharing manner, that is, the medical data is transmitted randomly through the two or more data connections. Of course, a hot backup manner may also be used to carry the medical data, that is, when one connection is faulty, another normal connection is used to transmit the medical data.

Further, healthy heartbeat detection is established between the front end processor and the data gateway. When a heartbeat of a connection is faulty, the front end processor automatically re-establishes a communication connection to the data gateway. If the communication connection fails to be established, an alarm is generated to notify a control scheduling of closing a connection. After all connections are closed, a medical institution server should be notified.

A difference between this embodiment and the prior art is that the connection managing module of the front end processor acquires the destination data gateway from the two or more data gateways according to a hot backup manner or a manner of alternately sharing information traffic and establishes a connection to a selected destination data gateway, so as to transmit the medical data, thereby ensuring a reliable connection between the front end processor and the data gateway and guaranteeing stability of the health information system.

(3) The two or more data gateways 103 are configured to receive the medical data and send the medical data to the data exchange platform.

The data gateway directly implements interworking between the platform and an information system of the medical institution and performs protocol interconnection of data exchange. A used protocol is the HL7/CDA specification. One data gateway on a regional health information platform may interconnect with multiple medical institutions.

The data gateway is specifically configured to be responsible for maintaining configuration information and connection states of a medical institution and a front end processor that are connected to the data gateway, so as to assist the control scheduling in summarizing and delivering a data packet. When the medical data sent by the front end processor is received, the data format of the medical data is parsed from the external protocol into the internal data packet format and the internal data packet format is assembled into the external protocol format. The data gateway is further responsible for interworking with the data exchange platform through a data exchange platform interface, delivering a protocol packet to a front end processor of a relevant medical institution according to configurations and running states of the medical institution and the front end processor, summarizing the received medical data, and forwarding it to the data exchange platform.

(4) The data exchange platform 104 is configured to process the received medical data.

The data exchange platform completes data exchange between medical institutions. Data exchange implements service collaboration. For example, if a patient is transferred from a hospital 1 to a hospital 2, the hospital 2 may query result information of an inspection and a check that are performed on the patient in the hospital 1 and like information from the hospital 1. A database may be configured for the data exchange platform. The database is configured to store and manage medical data within a region.

According to the system provided by this embodiment, multiple data gateways are disposed in a health information system, so that the health information system is capable of supporting multiple connections between a front end processor and the data gateways, thereby preventing a service from being affected due to congestion of a single data gateway in busy hours, improving data stability, and ensuring a normal medical service.

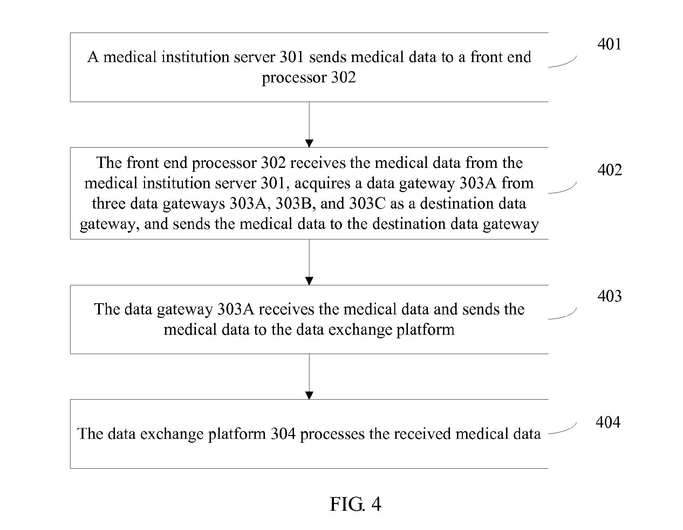

To describe a data processing method of a health information system provided by the present invention, this embodiment uses a system including three data gateways 302A, 302B, and 302C as an example for description. FIG. 3 is a schematic structural diagram of a health information system according to an embodiment of the present invention. Referring to FIG. 3, the system specifically includes: a medical institution server 301, a front end processor 302, three data gateways 303A, 303B, and 303C, and a data exchange platform 304. FIG. 4 is a flowchart of a data processing method based on the health information system in FIG. 3 according to the present invention. Referring to FIG. 3, the method includes:

401: The medical institution server 301 sends medical data to the front end processor 302.

402: The front end processor 302 receives the medical data from the medical institution server 301, acquires the data gateway 303A from the three data gateways 303A, 303B, and 303C as a destination data gateway, and sends the medical data to the destination data gateway.

Specifically, for this step, the front end processor 302 receives the medical data from the medical institution server 301, converts a data format of the medical data into a data format required by the data exchange platform 304, acquires the destination data gateway 303A from the data gateways 303A, 303B, and 303C, establishes a connection between the front end processor 302 and the destination data gateway 303A, and sends the medical data to the destination data gateway 303A through the established connection.

In this embodiment, that the front end processor 302 randomly acquires one data gateway as the destination data gateway is used only as an example for description. However, the front end processor 302 may further determine the destination data gateway by using any one of the following methods: (a) randomly acquiring one data gateway from the data gateways 303A, 303B, and 303C as the destination data gateway; (b) acquiring, according to a preset sequence, one data gateway from the data gateways 303A, 303B, and 303C as the destination data gateway; (c) using the data gateways 303A, 303B, and 303C as the destination data gateway; and (d) determining whether an active data gateway in the data gateways 303A, 303B, and 303C runs normally, and acquiring the active data gateway as the destination data gateway if the active data gateway runs normally; and acquiring a standby data gateway in the two or more data gateways as the destination data gateway if the active data gateway runs abnormally.

Because only one data gateway is selected as the destination data gateway in this embodiment, no traffic sharing problem is involved. In another embodiment, when the number of destination data gateways determined by the front end processor 302 is two or more, the medical data is sent to each destination data gateway. Further, or any one of the two or more destination data gateways classifies the medical data according to a user identifier of each piece of medical data and sends a same type of medical data to a same destination data gateway.

403: The data gateway 303A receives the medical data and sends the medical data to the data exchange platform.

404: The data exchange platform 304 processes the received medical data.

This embodiment uses that a medical institution server sends medical data to a data exchange platform only as an example for description. When a data exchange platform sends medical data to a medical institution server, the medical data may be returned according to a source or a requester of the medical data.

According to the method provided by this embodiment, multiple data gateways are disposed in a health information system, so that the health information system is capable of supporting multiple connections between a front end processor and the data gateways and load sharing-based access can be supported between the data gateways, thereby preventing a service from being affected due to congestion of a single data gateway in busy hours. A data exchange platform is capable of receiving access to information of each medical institution server in a balanced manner and each medical institution server accesses a regional health information platform by using multiple connections. In this way, a medical collaboration service may not be interrupted when a channel is faulty, thereby improving data stability and ensuring a normal medical service.

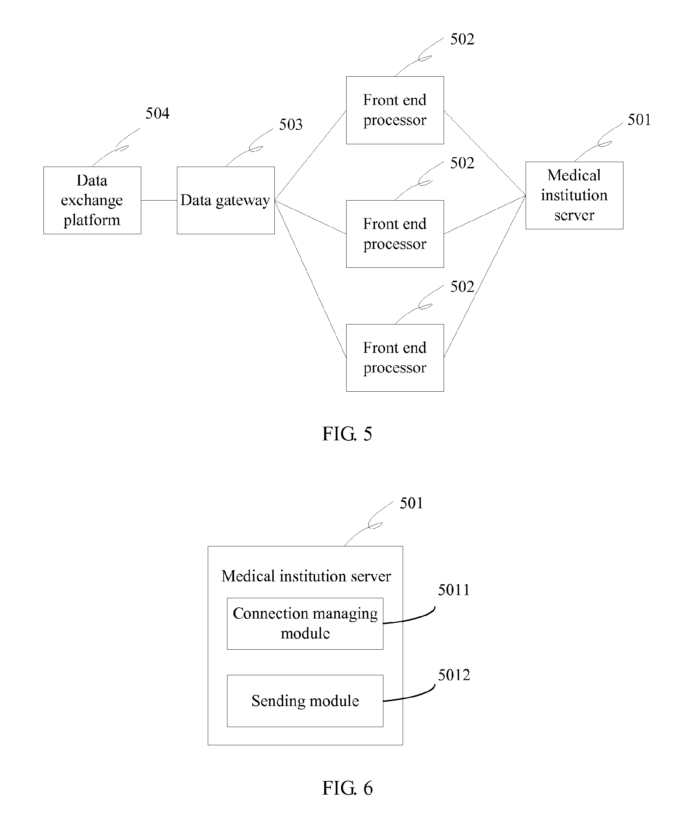

FIG. 5 is a schematic structural diagram of a health information system according to an embodiment of the present invention. Referring to FIG. 5, the system specifically includes at least one medical institution server 501, two or more front end processors 502, a data gateway 503, and a data exchange platform 504.

(1) The at least one medical institution server 501 is configured to acquire a destination front end processor from the two or more front end processors 502 and send medical data to the destination front end processor.

Preferably, referring to FIG. 6, the medical institution server includes: a connection managing module 5011, configured to acquire the destination front end processor from the two or more front end processors 502 and establish a connection to the front end processor; and a sending module 5012, configured to send the medical data to the destination front end processor through the established connection.

The connection managing module 5011 includes: a detecting unit, configured to detect a connection to any one of the two or more front end processors; and a switching unit, configured to, when the connection to the any one of the two or more front end processors is faulty, acquire the destination front end processor from a front end processor that is not faulty and establish a connection to the destination front end processor.

In this embodiment, that a method for a medical institution server to select a front end processor is failover is used only as an example for description. However, there may be multiple methods for a medical institution server to select a front end processor, for example, in a random manner or an alternate manner according to a preset sequence. Accordingly, medical data may also be sent by using multiple methods, for example, load sharing or failover.

(2) The two or more front end processors 502 are configured to receive medical data from the at least one medical institution server 501 and send the medical data to the data gateway 503.

In case that a health information system has two or more front end processors and a data exchange platform has only one data gateway, the two or more front end processors are connected to the data gateway separately. In this way, two or more connections exist. In this embodiment, the two or more connections undertake data communication in a load sharing manner. When one connection is faulty, a communication task is undertaken by another normal connection.

(3) The data gateway 503 is configured to receive the medical data and send the medical data to the data exchange platform 504.

The data gateway is specifically configured to be responsible for maintaining configuration information and connection states of a medical institution and a front end processor that are connected to the data gateway, so as to assist a control scheduling in summarizing and delivering a data packet. When the medical data sent by the front end processor is received, a data format of the medical data is parsed from an external protocol into an internal data packet format and the internal data packet format is assembled into an external protocol format. The data gateway is further responsible for interworking with the data exchange platform through a data exchange platform interface, delivering a protocol packet to a front end processor of a relevant medical institution according to configurations and running states of the medical institution and the front end processor, summarizing the received medical data, and forwarding it to the data exchange platform.

(4) The data exchange platform 504 is configured to process the received medical data.

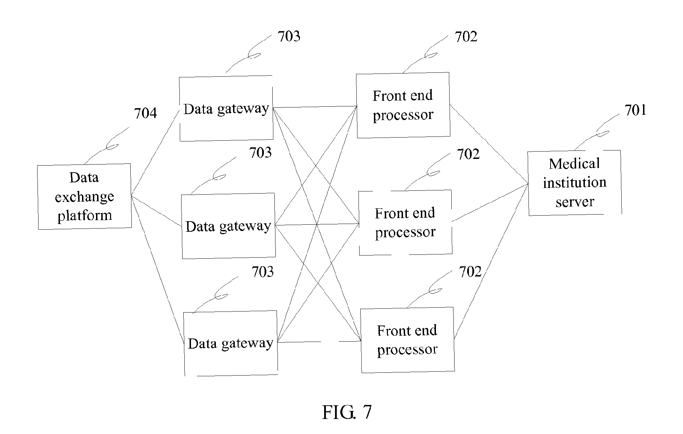

This embodiment provides a health information system. The health information system includes two or more front end processors. In another embodiment, referring to FIG. 7, the health information system may further include a medical institution server 701, two or more front end processors 702, two or more data gateways 703, and a data exchange platform 704. When the medical institution server 701 sends a data request to a front end processor 702, the front end processor 702 selects a connection in a normal state to send a medical data request to the data exchange platform 704. The data exchange platform 704 generates multiple pieces of medical data, and the data exchange platform 704 separately sends the medical data to the multiple data gateways 703 according to a policy. The data gateways 703 convert the medical data into a data format required by the medical institution server and send the medical data to the multiple front end processors 702 of the medical institution server through multiple connections in the normal state. The front end processors 702 summarize the multiple pieces of medical data and submit it to the medical institution server 701. When the data exchange platform 704 pushes the medical data to the medical institution server 701, the data exchange platform 704 generates the medical data and the data exchange platform 704 separately sends the medical data to the multiple data gateways 703 according to the policy. The data gateways 703 convert the medical data into the data format required by the medical institution server 701, and the data gateways 703 send the medical data to the multiple front end processors 702 of the medical institution server 701 through the connections in the normal state. The front end processors 702 submit the medical data to the medical institution server 701.

According to the system provided by this embodiment, multiple front end processors are disposed in a health information system, so that the health information system is capable of supporting multiple connections between the front end processors and a data gateway, thereby preventing a service from being affected due to congestion of a single data gateway in busy hours or a service from being affected due to a fault of a front end processor of a medical institution, improving data stability, and ensuring a normal medical service.

A person of ordinary skill in the art may understand that all or a part of the steps of the embodiments may be implemented by hardware or a program instructing relevant hardware. The program may be stored in a computer readable storage medium. The storage medium may include: a read-only memory, a magnetic disk, or an optical disc.

The foregoing descriptions are merely exemplary embodiments of the present invention, but are not intended to limit the present invention. Any modification, equivalent replacement, and improvement made without departing from the spirit and principle of the present invention shall fall within the protection scope of the present invention.

## Claims

1. A health information system, comprising at least one medical institution server, a front end processor, two or more data gateways, and a data exchange platform, wherein:
the at least one medical institution server is configured to send medical data to the front end processor;
the front end processor is configured to receive the medical data from the at least one medical institution server, acquire a destination data gateway from the two or more data gateways, and send the medical data to the destination data gateway;
the two or more data gateways are configured to receive the medical data and send the medical data to the data exchange platform; and
the data exchange platform is configured to process the received medical data.

2. The system according to claim 1, wherein the front end processor comprises:
a receiving module, configured to receive the medical data from the at least one medical institution server;
a data processing module, configured to convert a data format of the medical data into a data format required by the data exchange platform;
a connection managing module, configured to acquire the destination data gateway from the two or more data gateways and establish a connection between the front end processor and the destination data gateway; and
a sending module, configured to send the medical data to the destination data gateway through the established connection.

3. The system according to claim 1 or 2, wherein the connection managing module specifically comprises:
a destination data gateway determining unit, configured to acquire one or more data gateways from the two or more data gateways as the destination data gateway; and
a connection establishing unit, configured to establish the connection between the front end processor and the destination data gateway.

4. The system according to claim 3, wherein:
the destination data gateway determining unit is specifically configured to randomly acquire one data gateway from the two or more data gateways as the destination data gateway;
or,
the destination data gateway determining unit is specifically configured to acquire, according to a preset sequence, one data gateway from the two or more data gateways as the destination data gateway;
or,
the destination data gateway determining unit is specifically configured to use the two or more data gateways as the destination data gateway;
or,
the destination data gateway determining unit is specifically configured to determine whether an active data gateway in the two or more data gateways runs normally; and acquire the active data gateway as the destination data gateway if the active data gateway runs normally; and acquire a standby data gateway in the two or more data gateways as the destination data gateway if the active data gateway runs abnormally.

5. The system according to claim 3, wherein when the number of determined destination data gateways is two or more, the sending module is specifically configured to send the medical data to each destination data gateway or any one of the two or more destination data gateways.

6. The system according to claim 5, wherein the sending module is specifically configured to classify the medical data according to a user identifier of each piece of medical data and send a same type of medical data to a same destination data gateway.

7. A health information system, comprising at least one medical institution server, two or more front end processors, a data gateway, and a data exchange platform, wherein:
the at least one medical institution server is configured to acquire a destination front end processor from the two or more front end processors and send medical data to the destination front end processor;
the two or more front end processors are configured to receive the medical data from the at least one medical institution server and send the medical data to the data gateway;
the data gateway is configured to receive the medical data and send the medical data to the data exchange platform; and
the data exchange platform is configured to process the received medical data.

8. The system according to claim 7, wherein the medical institution server comprises:
a connection managing module, configured to acquire the destination front end processor from the two or more front end processors and establish a connection to the front end processor; and
a sending module, configured to send the medical data to the destination front end processor through the established connection.

9. The system according to claim 7, wherein the connection managing module comprises:
a detecting unit, configured to detect a connection to any one of the two or more front end processors; and
a switching unit, configured to, when the connection to the any one of the two or more front end processors is faulty, acquire the destination front end processor from a front end processor that is not faulty and establish a connection to the destination front end processor.
